# EUROPÄISCHE PATENTSCHRIFT

(11) **EP 1 877 535 B1**
(45) Veröffentlichungstag und Bekanntmachung des Hinweises auf die Patenterteilung: **12.06.2019**
(21) Anmeldenummer: 06724536.5
(22) Anmeldetag: 24.04.2006
(51) Int. Cl.: C12M 1/34, C12M 1/36

(54) **ZELLKULTURSYSTEM SOWIE VERFAHREN ZUR KULTIVIERUNG EINER ZELLKULTUR**
CELL CULTURE SYSTEM AND METHOD OF CULTIVATING A CELL CULTURE
SYSTEME DE CULTURE CELLULAIRE, ET PROCEDE DE CULTURE CELLULAIRE

(30) Priorität: 06.05.2005 DE 102005021034
(43) Veröffentlichungstag der Anmeldung: 16.01.2008
(73) Patentinhaber: Fraunhofer-Gesellschaft zur Förderung der angewandten Forschung e.V., 80686 München (DE)
(72) Erfinder: BRANDENBURG, Albrecht, 79104 Freiburg (DE); THIELECKE, Hagen, 66440 Blieshostel (DE); HOFFMANN, Christian, 79115 Freiburg (DE); MALTHAN, Dirk, 89073 Ulm (DE); SCHWARZ, Ron, 40591 Düsseldorf (DE)
(74) Vertreter: Grünecker Patent- und Rechtsanwälte PartG mbB
(86) Internationale Anmeldenummer: PCT/EP2006/003760
(87) Internationale Veröffentlichungsnummer: WO 2006/119860

(56) Entgegenhaltungen:
- EP-A- 1 270 718
- EP-A- 1 548 099
- EP-A- 1 612 262
- WO-A-2005/001108
- WO-A-2006/005808
- DE-A- 10 128 810
- US-A- 4 179 339

## Beschreibung

Die vorliegende Erfindung betrifft ein Verfahren zur Kultivierung einer Zellkultur in einem automatisierten Zellkultursystem und ein automatisiertes Zellkultursystem. Insbesondere betrifft die vorliegende Erfindung eine Vorrichtung und ein Verfahren zur Regelung von Zellkulturbedingungen und Zellkultivierungsprozeßschritten in Abhängigkeit vom Zustand der biologischen Zellen.

Zellkulturen werden in der Pharma- und Biotechnologieindustrie vielfach verwendet. Insbesondere aus ethischen und ökonomischen Gründen werden Tierversuche in den letzten Jahren verstärkt durch Zellkultursysteme und Zellkulturtechniken ersetzt. So empfehlen beispielsweise auch die Europäische Union und die OECD den Ersatz von Tierversuchen durch tierversuchsfreie Zelltests für die Prüfung von Arzneimitteln, Chemikalien und Kosmetika. Der Einsatz von Zellkulturen bei der Suche nach neuen Wirkprinzipien und Wirkstoffen auf dem Sektor der Pharmazeutika und der Pflanzenschutzmittel ist daher mittlerweile unverzichtbar.

Außerdem wird eine Reihe von in vitro Testmethoden im Bereich der Toxikologie vermehrt in behördliche Zulassungsverfahren aufgenommen. Somit wird auch in den Sektoren Pharmakologie und Pflanzenschutzmittel nicht mehr auf die Verwendung von Zellkulturen verzichtet.

Allerdings wird eine Aussagekraft der an Zellkulturen gewonnenen Daten derzeit noch durch die vorhandene Analysetechnik begrenzt. Außerdem sind gängige manuelle Methoden in der Zellkultivierung zeitaufwendig und kostenintensiv und ihre Ergebnisse sind zudem stark benutzerabhängig.

Aufgrund dessen wurden in jüngster Zeit Initiativen gestartet, um Mindestanforderungen an Zell- und Gewebekulturen zu definieren und damit die Aussagekraft, die Vergleichbarkeit und die Reproduzierbarkeit von in vitro Arbeiten zu sichern. Dies führt dazu, daß analog zu den Good Laboratory Practice (GLP)-Richtlinien, Grundsätze für Zellkulturarbeiten unter der Bezeichnung Good Cell Culture Practice (GCCP) erlassen wurden.

Diese Richtlinien haben jedoch keine Auswirkungen darauf, daß heute in der Regel immer noch Zellkulturen mit sehr hohem Einsatz von Laborpersonal kultiviert werden. Die Kultivierung der Zellen erfolgt dabei in der Regel in Petrischalen, Mikrotiterplatten oder in Zellkulturflaschen. In den meisten Fällen werden die zur Zellkultur notwendigen Schritte, wie Animpfen der Zellen, Wechseln des Mediums oder Passage der Zellen rein manuell durchgeführt. Außerdem erfolgt die Überwachung der Zellen diskontinuierlich. Änderungen des PH-Wertes des Mediums werden durch zugegebene Farbindikatoren überwacht. Das Medium wird in regelmäßigen Zeitabständen, abhängig von der Wachstumsrate der jeweiligen Zellen der Zellkulturen gewechselt (durch manuelle Kontrolle nach Entnahme aus einem Brutschrank), um eine ausreichende Versorgung der Zellen mit Nährstoffen zu gewährleisten.

Da aus den Ergebnissen der Zellkultivierung, d.h. aus der Entwicklung der Zellkultur unter dem Einfluß bestimmter Faktoren, Rückschlüsse auf die Wirksamkeit von Medikamenten oder auf eine toxische Wirkung von Substanzen (z.B. in Kosmetika) gezogen werden können, hat die reproduzierbare Kultivierung und der standardisierte Betrieb der Zellkultur besondere Bedeutung für die genannten Anwendungsfelder.

Die Standardisierbarkeit der Zellkulturtechniken ist wegen dieser hohen Abhängigkeit von manuellen (und damit benutzerabhängigen und nicht reproduzierbaren) Methoden und des daraus resultierenden hohen Aufwandes bisher unzureichend für die Verwendung dieser Kultur in Anwendungen wie der Stammzellenforschung, dem Screening oder dem Tissue Engineering.

Voraussetzung hierfür sind vielmehr standardisierte Zellkulturverfahren, da nur diese über Jahre hinweg einen Vergleich zwischen den Substanzen und ihren Wirkprinzipien ermöglichen.

Im Einzelnen bestehen dementsprechend die folgenden Probleme:
Die Anpassung der Zellkulturbedingungen erfolgt bei bestehenden Lösungen auf Grund fester Protokolle und dadurch starren Abläufen im Zellkultivierungsprozess, wobei diese Protokolle auf der manuellen Kultivierung durch das Laborpersonal und deren Erfahrungswerten sowie dem subjektiven Empfinden des Operators und den eingeschliffenen Tagesabläufen im Zellkulturlabor beruhen.

Die Kontrolle und Dokumentation des Zellzustandes erfolgt aus Aufwandsgründen normalerweise nur im Stunden- oder Tagesabstand. Dies führt zu langen Kontrollintervallen. Bei der Kultivierung sensibler Zellen, wie insbesondere von Stammzellen, ist es oft jedoch notwendig, die Zellen in kürzeren Abständen zu kontrollieren.

Bei der Charakterisierung der Zellen erfolgt die Auswahl der untersuchten Bereiche subjektiv und zufällig durch den Operator. Dabei sind selbst Softwareunterstützungen für die optische Analyse insoweit manuell und nicht reproduzierbar.

Beim manuellem Wechsel der Zellkulturmedien kommt es zu individuellen Zeitschwankungen und dadurch zu Schwankungen bei der Versorgung der Zellen. Ebenfalls kommt es zu Streß für die Zellen, beispielsweise auf Grund undefinierter Strömungen.

Bei der Charakterisierung der Zellen durch den Operator kommt es zudem zur Beeinflussung der Zellen auf Grund starker Schwankungen der Zellkulturbedingungen, beispielsweise Änderungen der Umgebungstemperatur von >10K, also zu einer Unterbrechung der Kulturbedingungen. Deren Auswirkung auf die Zellentwicklung ist nicht vorhersagbar.

Weiterhin besteht die Gefahr von Kontaminationen durch eine Vielzahl manueller Prozesse sowie durch Automatisierungslösungen mit geringer hygienischer Qualität.

Nachteilig ist außerdem, daß die Entscheidung des nächsten Schrittes in dem Zellkultivierungsprozeß nach subjektiver Beurteilung (beispielsweise abhängig vom Operator, vom Wochentag bzw. der Tageszeit, oder von Bildgebungseinstellungen des Mikroskops) der Zellkultur (beispielsweise Bewertungen wie "Konfluenzgrad ca. 70 oder 80 %", "Medium ungefähr verbraucht" oder "Zellen während der Passage nun ungefähr alle abgelöst") und nach Planung des Zellkultivierungspersonals bzw. dessen Arbeitszeit (es wird beispielsweise als ausreichend betrachtet, wenn "morgen" oder "am Montag nach dem Wochenende" oder "nach den Feiertagen" passagiert bzw. ein Medienwechsel vorgenommen wird und das Personal/der Operator daher nicht außerhalb seiner regulären Arbeitszeit den Zellkultivierungsprozess ausführen muss).

Bei am Markt bekannten Systemen werden Zellkulturkammern als autarke Systemkomponenten neben Automatisierungssystemen verwendet, wobei die Automatisierungssysteme beispielsweise ausschließlich für die Passage oder den Medienwechsel verwendbar sind. Die Automatisierungssysteme sind dabei kein integraler Teil des Zellkulturprozesses, da die verfügbaren Systeme z. B. Zellaussaat, Waschschritte, Mediumwechsel und Zellernte eigenständig durchführen. Je nach System können auch weitere Schritte wie Transfektion oder Zellzahlbestimmung ausgeführt werden.

Bei der Charakterisierung von Zellen durch biochemisch-molekularbiologische Methoden können derzeit nur Momentaufnahmen erhalten werden, da die Zellen durch die Darstellungsmethoden häufig abgetötet werden. Die entsprechenden Meßmethoden sind zeitaufwendig und nicht geeignet, Zellen über einen längern Zeitraum zu überwachen. Eine andauernde und lückenlose Kontrolle und Überwachung der Zellen ist mit den vorhandenen Techniken nicht möglich.

Die Kulturbedingungen für die Zellen sind vielfach durch Erfahrungswerte geprägt, so z.B. die Zeitabstände, wann das Kulturmedium gewechselt wird. Dies erschwert eine mögliche Standardisierung der Zellkulturen.

Demgegenüber wären Systeme vorteilhaft, die in der Lage sind, eine regelmäßige Überwachung der Zellen in kurzen Zeitabständen durchzuführen. Darüber hinaus müssten auf Grund der erfassten Daten die Kultivierungsbedingungen nachgeregelt werden, falls dies notwendig ist.

Auf dem Markt befindliche Bildanalysesoftware für die Analyse von Zellmikroskopiebildern ist entweder auf sehr spezielle Aufgaben abgestimmt (z.B. Zählung von gefärbten Zellen in Suspensionen) oder sie erfordert sehr gute Bildverarbeitungskenntnisse beim Anwender, um die Algorithmen und Parameter genau an die jeweilige Analyse-Aufgabe anzupassen. Bei der Bildanalyse von Zellkulturen ist eine solche Anpassung zwingend erforderlich, da verschiedene Zelltypen sehr unterschiedlich aussehen können und auch Zellen eines Typs je nach Kultivierungsstadien stark variieren. Allerdings haben Anwender in der Biologie in der Regel nicht den notwendigen technischen Hintergrund, um eine entsprechend richtige Einstellung der Software vornehmen zu können.

Ein Verfahren zur Kultivierung einer Zellkultur ist aus der EP 1 612 262 A1 bekannt. Hierbei ist es jedoch unverändert erforderlich, die Kulturen durch den Anwender mikroskopisch hinsichtlich der Wachstumsbedingungen innerhalb des Kulturbehälters zu beobachten oder die Anzahl der Zellen zu zählen.

Ferner ist aus EP 1 205 742 A2 eine Einrichtung zur automatisierten Klassifikation zellulärer Strukturen bekannt, die allerdings nur kleine Substrukturen in Fluoreszenzbildern erkenn kann, wobei die eingesetzte Software eine Vorauswahl der zu untersuchenden Bereiche hinsichtlich einer Fluoreszenz benötigt.

Dokument WO2005001108 offenbart eine computergestützte, digitale Bildverarbeitungseinrichtung zur kontinuierlichen Überwachung für eine kontinuierliche Steuerung oder Regelung zur Optimierung des Kultivierungsprozesses.

Ausgehend hiervon ist es eine Aufgabe der vorliegenden Erfindung, ein Verfahren zur Kultivierung einer Zellkultur in einem automatisierten Zellkultursystem und ein automatisiertes Zellkultursystem anzugeben, bei denen die Automatisierung Teil des Zellkulturprozesses ist, um optimierte und standardisierte Zellkulturbedingungen zu erreichen.

Die Aufgabe wird verfahrensseitig erfindungsgemäß durch ein Verfahren nach Anspruch 1 gelöst.

In vorrichtungstechnischer Hinsicht wird die Aufgabe erfindungsgemäß gelöst durch ein automatisiertes Zellkultursystem mit den Merkmalen des Anspruchs 4.

Bei dem erfindungsgemäßen Verfahren und dem erfindungsgemäßen Zellkultursystem werden optimierte und standardisierte Zellkulturbedingungen dadurch erreicht, dass die Kultur in einem automatisierten Zellkultursystem gehalten wird, das (Zustands-)Daten der Zelle erfasst, und zudem abhängig von deren Zustand die Zellkulturbedingungen (vorzugsweise unmittelbar) nach Erfassung des Zustandes der Zellkultur regelt und/oder notwendige Prozessschritte in der Zellkultivierung einleitet/einsteuert. Damit wird der Einfluss des menschlichen Operators reduziert und durch eine präzisiere automatische Lösung ersetzt. Es wird außerdem eine reproduzierbare Vorgehensweise implementiert, die den natürlichen Schwankungen der lebenden Zellkulturen Rechnung trägt.

Bevorzugte Ausführungsbeispiele des erfindungsgemäßen Verfahrens und des erfindungsgemäßen automatisierten Zellkultursystems sind Gegenstand der abhängigen Ansprüche.

Die vorliegende Erfindung wird nachfolgend anhand bevorzugter Ausführungsbeispiele in Verbindung mit der zugehörigen Zeichnung näher erläutert, in der eine optische Anordnung zur Gewinnung von quantifizierbaren räumlichen Informationen der Zellen und Zellkulturen gezeigt ist.

Ein bevorzugtes Ausführungsbeispiel des automatisierten Zellkultursystems weist als aktorische Elemente eine automatisierte Handhabungseinheit zum Transport von Zellkulturbehältern (beispielsweise Zellkulturflaschen oder Mikrotiterplatten), eine automatisierte Handhabungseinrichtung für Flüssigkeiten (beispielsweise der Zellsuspensionen, Fluoreszenzfarbstoffe und Nährmedien) und einen Speicher für zeitweilig nicht verwendete Zellkulturgefäße auf.

Das vorliegende automatisierte Zellkultursystem weist zudem eine Klimatisierungseinheit zur Herstellung der äußeren Zellkulturbedingungen, insbesondere eine klimatisierte Vertikalumluftbank mit definierten Umgebungsbedingungen, auf.

Das vorliegende Ausführungsbeispiel weist weiterhin eine Einheit zur Erzeugung eines Laminar Flow, eine Medienzuführung, Vorrichtungen zur Sterilisation von Oberflächen und eine Zentrifuge auf. Durch eine entsprechende Gestaltung der Oberflächen sowie die Auswahl des Materials wird hierbei eine Dekontaminierbarkeit erzielt, wodurch die Gefahr von Querkontaminationen minimiert wird. Hierzu zählt beispielsweise die Vermeidung von ungedichteten Schraubverbindungen, von Toträumen oder ebenen Konstruktionsflächen, die einen Abfluß von evtl. Spülflüssigkeiten oder Produktionsrückständen verhindern würden.

Außerdem wird der Zustand der Zellen in der Zellkultur im vorliegenden Ausführungsbeispiel des automatisierten Zellkultursystems mit einer oder mehrerer Vorrichtungen zur Gewinnung von Informationen aus der Kultur erfaßt. Hierzu sind (als sensorische Elemente) eine optische Bilderfassung und/oder elektrische/elektrochemische Sensoren vorgesehen.

Beispielsweise ist eine optische Untersuchung der Zellkultur mit verschiedenen bildgebenden mikroskopischen Verfahren, wie Durchlicht, Phasenkontrast oder differentieller Interferenzkontrast ,DIC' möglich.

Gegebenenfalls kommt eine bildgebende Fluoreszenzoptik hinzu, mit der die räumliche Verteilung von Fluoreszenzfarbstoffen in der Zellkultur bestimmt wird.

Diese bildgebenden Verfahren sind an eine automatische Bildauswertung angeschlossen, welche geeignet ist, Rückschlüsse auf den Zustand einzelner Zellen oder der gesamten Kultur zu ziehen. Dabei ergibt die räumliche Struktur der Zellen und Zellkulturen eine interessante zusätzliche Information.

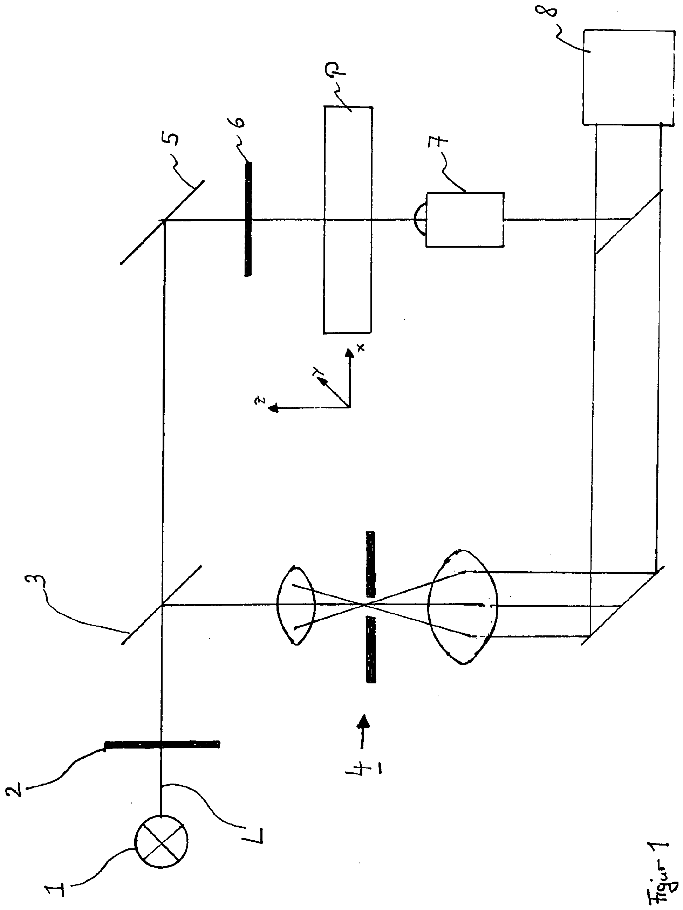

Zur Gewinnung derartiger quantifizierbarer Informationen über eine räumliche Struktur der Zellen und Zellkulturen sind die konventionellen Methoden wie Phasenkontrast und differentieller Interferenzkontrast (DIC) jedoch ungeeignet. Diese ergeben zwar visuell einen Eindruck über die dritte Dimension (in der Beobachtungsrichtung, z-Richtung in der Figur). Diese Information ist jedoch nicht in allen Fällen eine quantitative Information, die in einer Bildauswertungssoftware weiterverarbeitet werden kann. Insbesondere sind die Bilder, die mit den genannten konventionellen Kontrastverfahren gewonnen werden, stark abhängig von der Einstellung der Optik.

Eine objektive, quantifizierbare Information erhält man dagegen durch ein interferometrisches Verfahren, bei welchem zusätzlich zu dem mikroskopischen Strahlengang (Meßstrahlengang) ein zusätzlicher Referenzstrahlengang hinzugefügt ist.

In der (einzigen) Figur ist eine derartige optische Anordnung zur Gewinnung quantifizierbarer räumlicher Informationen über die Zellen und Zellkulturen gezeigt.

Dabei wird von einer Lichtquelle 1 ein Lichtstrahl L erzeugt, welcher über einen Polarisationsfilter 2 geführt wird. Der polarisierte Strahl mit einer Wellenlänge von λ/2 wird mittels eines Strahlteilers 3 auf zwei Teilstrahlen L1, L2 aufgeteilt. Der Teilstrahl L1, welcher den Referenzstrahl bildet, durchläuft eine Baugruppe aus einem Teleskop und einem Raumfilter (mit dem Bezugszeichen 4 bezeichnet). Der Teilstrahl L2, der den Meßstrahl bildet, wird über eine Umlenkeinrichtung 5 und einen weiteren Polarisationsfilter 6 der Probe P zugeführt. Nach Durchsetzen der Probe P durchläuft der Meßstrahl L2 ein Mikroskopobjektiv 7. Der das Mikroskopobjektiv 7 verlassende Meßstrahl wird auf einer Auswerteoptik 8 (insbesondere einem CCD-Feld) mit dem Referenzstrahl L1 überlagert.

Die Strahlaufteilung auf die Strahlteilen L1 und L2 erfolgt vorliegend gleichmäßig, d.h. die Intensität L1 zu L2 beträgt bei 50 % zu 50 %.

Wie in der (einzigen) Figur ersichtlich, wird der Referenzstrahl L1 mit dem aus dem Objektiv austretenden Meßstrahl L2 überlagert. Das auf der optischen Auswerteinrichtung 8 entstehende Interferenzmuster enthält die Information über die Phasenverschiebung des Lichtes an jedem Ort in der x-y-Ebene auf der Probe (in der Figur ist ein kartesisches Koordinatensystem angegeben). Die optische Phase ist gleichbedeutend mit dem Produkt aus Dicke und Brechungsindex des beobachteten Objektes P. Unter der Annahme, daß die Brechzahlen innerhalb der Zellen homogen und von Zelle zu Zelle identisch sind, ist daraus die Dicke der Zellen an jedem Ort in der x-y-Ebene berechenbar.

Zur eindeutigen Bestimmung der Phase wird beim vorliegenden Ausführungsbeispiel eine Komponente (Teleskop + Raumfilter) zur definierten Phasenverschiebung des Referenzstrahles eingeführt. Aus mehreren Bilder, die mit verschiedenen Referenzphasen aufgenommen wurden, wird eindeutig zwischen Phasen- und Intensitätsinformation unterschieden. Gleichzeitig erhält man einen innerhalb eines Bereiches von 2π eindeutigen Phasenwert. Über eine Interferenzordnung (Phasenänderung von 2π) hinaus ist die Phase mit Hilfe bekannter "unwrapping"-Techniken zu korrigieren.

Neben den vorgenannten bildgebenden Verfahren sind außerdem optische Verfahren von Bedeutung, mit denen die optische Transmission oder die Fluoreszenz über größere Bereiche der Kultur gemittelt erfaßt werden können. Damit kann man z.B. die Färbung von Indikatoren erfassen, die dem Kulturmedium zugesetzt werden. Hierdurch können Methoden zur Mittlung der Zelleigenschaften zur Verfügung gestellt werden.

Weiterhin können elektrische bzw. elektrochemische Meßverfahren zum Einsatz kommen, mit denen z.B. die lokale Temperatur, der pH-Wert oder lonenkonzentrationen bestimmt werden.

Um den Zustand der Zellkultur automatisch bestimmen zu können, ist eine Software für die Auswertung von Bilddaten und Sensorsignalen und zur Steuerung des gesamten Ablaufs der Zellkultivierung vorgesehen. Mit dieser werden die vom System erfaßten Bilddaten der kultivierten Zellen über eine Bildanalyse verarbeitet. Diese liefert quantitative Analyseergebnisse, wie beispielsweise Zelldichte, Zahl der toten Zellen, Zahl der Mitosen, und Zahl der morphologisch veränderten Zellen, welche ein Bild von der Kultur ergeben, das zusammen mit der Vorgeschichte, d.h. früher an dieser Kultur erfaßten Daten und zusammen mit den Protokollen zur Kultivierung der speziellen Kultur zu einer sehr genauen Bewertung des aktuellen Zustandes der Zellkultur führen können. Diese sehr genaue Bewertung des Zustandes der Zellkultur ist eine solide Grundlage für die Feststellung eines möglichen Eingriffs in die Kulturbedingungen und/oder für die Initiierung eines Prozeßschrittes in den Kultivierungsprozeß. Dieser Eingriff kann dann direkt durch das System erfolgen oder zu einer Meldung nach außen führen. Das System kann in diesem Fall eine Meldung, ggf. mit einem Vorschlag für eine Aktion machen, die dann vom Operator ausgelöst wird.

Die Verarbeitung der Zellbilder und der Sensorsignale ist erfindungsgemäß als trainierbare Software ausgeführt. Damit ist das System leicht an veränderte Aufgabenstellungen (wie andere Zellkulturen öder veränderte Rahmenbedingungen) anpaßbar. Der Nutzer gibt z.B. anhand von Bildern eine Klassifizierung von Zelltypen oder von definierten Zuständen der Kultur vor, die anschließend während der Kultivierung von der Bildverarbeitung erkannt werden.

Insbesondere kann dabei die Bildanalysesoftware für die Erkennung von relevanten Bildstrukturen, wie insbesondere gesunde Zellen, zertrümmerte Zellen, abgelöste Zellen durch den Anwender trainiert werden. Dazu werden in einer Trainingsphase in einigen Bildern Beispiele für zu erkennende Strukturen markiert. Die Bildanalysesoftware berechnet für diese Bilder einen Satz von Merkmalen (Intensität, morphologische Merkmale, Texturen) und bestimmt diejenigen Merkmale, welche die ausgewählten Beispiele auszeichnen, und insbesondere Kriterien, die im Merkmalsraum die gesuchten Strukturen von anderen Strukturen diskriminieren. Nach Abschluß der Trainingsphase können diese berechneten Parameter verwendet werden, um in weiteren Bildern die gewünschten Strukturen automatisch zu erkennen.

Neben der automatischen Datenerfassung und Regelung ist die Dokumentation (z.B. der Regelparameter, der Meßwerte der Regelung, des Zustands der Zellkultur bzw. des Mediums zu verschiedenen Zeitpunkten, der Meßwerte der Kultivierungsparameter und des Zustands der Zellkultur auf deren Basis eine Regelung oder ein Prozeßschritt durchgeführt wurde) von großer Bedeutung. Auf diesem Wege können die Vorgeschichte der Kultur und der Kultivierung zurückverfolgt werden. Damit wird eine zeitlich abhängige präzisere Zustandsbeschreibung gegeben und im Falle eines nicht optimalen Ergebnisses die Fehlersuche erleichtert.

Bei einem ersten Anwendungsbeispiel des vorliegenden automatisierten Zellkultursystems wird für eine optimale Zelldifferenzierung die Zusammensetzung des Differenzierungsmediums an den Differenzierungsgrad der Zellkultur angepaßt. Dazu werden kontinuierlich Bilddaten von der Zellkultur aufgenommen und mit Hilfe der Auswertesoftware ausgewertet. Bei bestimmten Zuständen der Zellen, die von der Software erkannt werden, erfolgt entweder ein automatischer Austausch des im Zellkulturgefäß vorhandenen Mediums durch ein neues Medium in anderer Zusammensetzung oder es werden zum vorhandenen Medium Wachstumsfaktoren zugegeben. Vorzugsweise kommt für die Bildanalyse die vorstehend beschriebene trainierbare Software zum Einsatz. Der Zustand der Zellkultur wird beispielsweise anhand von morphologischen Merkmalen oder mit Hilfe minimal invasiver Markierungssubstanzen erfaßt. Ein Trainieren der Bildanalysesoftware kann in Vorversuchen durch erfahrenes Personal oder auch mit Hilfe der invasiven Markierungssubstanzen erfolgen.

Bei einem zweiten Anwendungsbeispiel erfolgt der Austausch des Kulturmediums in Abhängigkeit von der Zellfunktion. Dabei wird quasi kontinuierlich eine bestimmte Zellfunktion, z.B. eine Kontraktionsrate bei Kardiomyozyten, überwacht. Sobald die Parameter der gewählten Zellfunktion einen zuvor definierten Bereich verlassen, wird ein Austausch des Zellkulturmediums veranlasst.

Bei einem dritten Anwendungsbeispiel wird die Kultivierung eines ersten Zelltyps mit Hilfe eines weiteren Zelltyps (Feederzellen) unterstützt. Wenn in der Mischkultur mit diesen beiden Zelltypen der erste Zelltyp in ausreichender Menge vorhanden ist, wird das vorhandene Kulturmedium automatisch durch ein Kulturmedium ersetzt, welches dazu führt, daß der zweite Zelltyp abstirbt. Wenn alle Zellen des zweiten Typs abgestorben sind, wird das Kulturmedium automatisch durch Normalmedium ersetzt.

Bei einem vierten Anwendungsbeispiel wird auf Grund eines Zustands der Zellkultur ein Prozeßschritt eingeleitet, wie z.B. Passage der Zellkultur bei Erreichen eines angestrebten Bedeckungs-/Konfluenzgrades in weitere Zellkulturflaschen oder andere Zellcontainer, Dispensieren der Zellen in Mikrotiterplattenwells oder Einzelzellenentnahme-/handling/-weiterverarbeitung.

Bei einem fünften Anwendungsbeispiel werden Zellen in Containern (z.B. Petrischalen, MTP, Zellkulturflaschen) kultiviert, Prüfsubstanzen (z.B. bei Wirkstofftest oder Toxizitätstests) aufgegeben und die Zellkultur in ihrer Eigenschaft (z.B. Wachstumsrate, Morphologie, Kern-Plasma-Verhältnis, Art des Wachstums wie beispielsweise Criss-cross-Wachstum, Ausbildung der Zellfunktion, Zellzahlbestimmung in bestimmten Arealen, beispielsweise Koloniemitte/Kolonierand) zeitabhängig dokumentiert und dem System ein Vorschlag für die Klassifikation (z.B. normal, verändert, transformiert, Zellzahl im Areal, mittlere Kerngröße, mittlere Zellplasmafläche, mittlere Neutritenanzahl etc.) gemacht.

Ein sechstes Anwendungsbeispiel betrifft die Optimierung des Zellkulturprozesses bei Primärzellkulturen, z.B. Keratinozyten aus unterschiedlichen Biopsien, und/oder den Erhalt der Zellkultur ohne weiteres Ausdifferenzieren.

Ein siebtes Anwendungsbeispiel betrifft einen autonomen Kultivierungsprozeß einer robusten Routinezellkultur (z.B. HeLa, HEK, COS) einschließlich Medienwechsel, Passage, Aussäen basierend auf der optischen und/oder elektrischen/elektrochemischen Sensorik (z.B. für Screeningexperimente mit Zellen gleicher Qualität innerhalb einer Zellkultur) unabhängig von Regelarbeitszeiten des Laborpersonals (z.B. über Nacht, über das Wochenende, über Feiertage).

Ein achtes Anwendungsbeispiel betrifft die Standardisierung einer Zellkultur (Definition der Prozeßparameter für die Zellkultivierung, z.B. Zeitpunkt für Passage oder Medienwechsel, Seeding Density, Planting Efficiency (Wieviele Zellen wachsen an?)) mit dem vorliegenden automatisierten Zellkultursystem auf Grund der schon vorstehend erläuterten Dokumentation, bevor dies in ein System mit starren Protokollen überführt wird oder die Kultivierung im Bioreaktor/Fermenter etabliert wird.

Bei einem neunten Anwendungsbeispiel werden bestimmte Bereiche aus einer Zellkultur entnommen, die sich während der Kultivierung gebildet haben und charakteristische Merkmale aufweisen. Sobald diese Bereiche entstanden sind, wird das mit Hilfe einer Bildanalyse erkannt und die Zellen der Bereiche automatisch mit Hilfe einer Pipette in ein neues Kulturgefäß überführt.

Bei einem zehnten Anwendungsbeispiel werden aus einer Zellsuspension mit sehr geringer Zellkonzentration Aliquote in Wells einer Mikrotiterplatte gegeben. In dem geregelten Zellkultursystem werden nur Zellen aus Wells weiterverarbeitet, in denen sich anfangs genau eine Zelle befand.

Aus der vorstehenden Beschreibung ergibt sich insbesondere ein automatisiertes Zellkultursystem, bestehend aus aktorischen Elementen (zur Handhabung von Zellkulturgefäßen, Zellsuspensionen, Fluoreszenzfarbstoffen und Nährmedien) einer klimatisierten Vertikalumluftbank mit definierten Umgebungsbedingungen, sensorischen Elementen (optische Bilderfassung und/oder elektrische Sensorik) und einer Software für die Auswertung von Bilddaten und Sensorsignalen und zur Steuerung des gesamten Ablaufes, wobei die Kulturbedingungen geregelt und/oder situationsabhängig Prozeßschritte in der Kultivierung initiiert und/oder durchgeführt werden.

Die Bildaufnahme erfolgt dabei u.a. mit einer interferometrischen Methode. Dabei wird zusätzlich zu dem mikroskopischen Strahlengang ein Referenzstrahl eingeführt, der mit dem Strahlenbündel überlagert wird, das aus dem Mikroskopobjektiv austritt.

Die Verarbeitung der Zellbilder und der Sensorsignale ist vorliegend als trainierbare Software ausgeführt. Damit ist das System leicht an veränderte Aufgabenstellungen (andere Zellkulturen, veränderte Rahmenbedingungen) anpaßbar.

Die Systemkomponenten sind in einer hohen hygienischen Qualität ausgeführt und verhindern dadurch die Kontamination des Produktes. Dies betrifft insbesondere die Gestaltung der Oberflächenrauhigkeit kleiner als 20 µm, entsprechende Dichtungselemente aus PTFE oder ähnlichen Materialien sowie eine entsprechende konstruktive Auslegung.

Die Planung der automatisierten Zellkultivierung erfolgt bezogen auf den gewünschten Endzeitpunkt der Produktion. Ausgehend von einem Zeitpunkt, zu dem die Zelllinien verfügbar sein sollen, wird eine rückwärtige Planung durchgeführt, wann Medienwechsel und Passagen durchgeführt werden sollen.

## Patentansprüche

1. a) Verfahren zur Kultivierung einer Zellkultur in einem automatisierten Zellkultursystem, wobei Zustandsdaten von Zellen in der Zellkultur erfasst und zumindest eine Kulturbedingung entsprechend dem erfassten Zustand der Zellen in der Zellkultur geregelt und/oder zumindest ein als notwendig ermittelter Prozessschritt in der Zellkultivierung eingeleitet oder durchgeführt wird, wobei an der Zellkultur erfasste Daten und/oder Protokolle zur Kultivierung zur Kultivierung der Zellen gespeichert werden und abrufbar sind,
b) eine Einrichtung zur Regelung von Zellkulturbedingungen vorgesehen ist, einen aktuellen Zustand der Zellkultur auf Basis der an der Zellkultur erfassten Daten und gespeicherter Protokolle früherer Kultivierungen zur Kultivierung der aktuellen Zellkulturen zu bewerten, wobei diese Bewertung Grundlage für eine Festlegung eines möglichen Eingriffs in die Kulturbedingungen ist,
c) zumindest ein Teil der Zellen in der Zellkultur mit einem bildgebenden optischen mikroskopischen Verfahren untersucht wird, und durch ein Auswertprogramm mindestens ein Parameter zur Kennzeichnung des Zustandes der Zellkultur ermittelt wird oder eine Klassifizierung der Zellen durchgeführt wird,
d) wobei zur Regelung der Kulturbedingungen auf zumindest einen Prozessparameter für die Zellkultivierung entsprechend der vom Auswerteprogramm vorgenommene Klassifizierung durch ein Regelungsprogramm Einfluss genommen wird und
die Zellkultur im automatisierten Zellkultursystem in einem Zellkulturbehälter mittels einer automatisierten Handhabungseinheit transportiert wird,
e) wobei ein Bildanalyseprogrammteil eines Softwareprogramms für die Erkennung von Zellen durch den Anwender trainierbar ist,
f) wobei in einer Trainingsphase einige der Bilder als Beispiele für zu erkennende Strukturen markiert werden, der Bildanalyseprogrammteil für diese Bilder einen Satz von Merkmalen berechnet und diejenigen Merkmale bestimmt, die die ausgewählten Beispiele auszeichnen sowie Kriterien bestimmt, die im Merkmalsraum die gesuchten Strukturen von anderen Strukturen unterscheiden, und wobei nach Abschluss der Trainingsphase diese berechneten Parameter verwendet werden, um in weiteren Bildern die gewünschten Strukturen automatisch zu erkennen und
g) wobei der Anwender anhand von Bildern eine Klassifizierung von Zelltypen oder von definierten Zuständen der Kultur vornimmt, die anschließend während der automatischen Kultivierung von einer Bildverarbeitung erkannt werden.

2. a) Automatisiertes Zellkultursystem mit zumindest einem aktorischen Element zur Handhabung einer Zellkultur, zumindest einem sensorischen Element zur Erfassung von Zustandsdaten der Zellen in der Zellkultur, einer Einrichtung zur Auswertung der Zustandsdaten der Zellen und einer Einrichtung zur Regelung von Zellkulturbedingungen und/oder zur Einleitung und/oder Durchführung weiterer Prozessschritte in der Kultivierung, zur Durchführung des Verfahrens nach Anspruch 1,
b) wobei als sensorisches Element eine optische mikroskopische Bilderfassung zur zwei- oder dreidimensionalen Darstellung der Zellen vorgesehen ist,
c) eine Speichereinrichtung, mit der an der Zellkultur erfasste Daten und/oder Protokolle zur Kultivierung der Zellkulturen speicherbar und abrufbar sind,
d) eine Einrichtung zur Regelung von Zellkulturbedingungen vorgesehen ist, einen aktuellen Zustand der Zellkultur auf Basis der an der Zellkultur erfassten Daten und gespeicherter Protokolle früherer Kultivierungen zur Kultivierung der aktuellen Zellkulturen zu bewerten, wobei diese Bewertung Grundlage für eine Festlegung eines möglichen Eingriffs in die Kulturbedingungen ist, und
e) wobei die Einrichtung zur Regelung von Zellkulturbedingungen dazu vorgesehen ist, einen Eingriff direkt im System mit den automatischen aktorischen Elementen zur Handhabung und zum Betrieb der Zellkultur automatisch durchzuführen und/oder eine Meldung nach außen zu geben,
f) wobei die Einrichtungen zur Auswertung der Zustandsdaten der Zelle und zur Regelung von Zellkulturbedingungen und/oder zur Einleitung und/oder Durchführung weiterer Prozessschritte als ein Software-Programm für die Auswertung von Bilddaten und zur Steuerung des gesamten Ablaufes ausgebildet sind,
g) wobei ein Bildanalyseprogrammteil des Softwareprogramms für die Erkennung von Zellen durch den Anwender trainierbar ist,
h) wobei in der Trainingsphase einige der Bilder als Beispiele für zu erkennende Strukturen markierbar sind, wobei der Bildanalyseprogrammteil für diese Bilder einen Satz von Merkmalen berechnet und diejenigen Merkmale bestimmt, die die ausgewählten Beispiele auszeichnen sowie Kriterien bestimmt, die die gesuchten Strukturen von anderen Strukturen unterscheiden, und wobei nach Abschluss der Trainingsphase diese berechneten Parameter verwendet werden, um in weiteren Bildern die gewünschten Strukturen automatisch zu erkennen, und
i) wobei ein Nutzer anhand von Bildern eine Klassifizierung von Zelltypen oder von definierten Zuständen der Kultur vornimmt, die anschließend während der automatischen Kultivierung von der Bildverarbeitung erkennbar sind.

3. Automatisiertes Zellkultursystem nach Anspruch 2, **dadurch gekennzeichnet, dass** das aktorische Element zum Handling der Zellkulturgefäße, der Zellsuspensionen, der Fluoreszenzfarbstoffe und/oder der Nährmedien vorgesehen ist.

4. Automatisiertes Zellkultursystem nach Anspruch 2 oder 3, **gekennzeichnet durch** eine klimatisierte Vertikalumluftbank mit definierten Umgebungsbedingungen.

5. Automatisiertes Zellkultursystem nach einem der Ansprüche 2 bis **4, dadurch gekennzeichnet, dass** als mikroskopisches Verfahren eine optische Bilderfassung, insbesondere Durchlicht, Phasenkontrast, oder differenzieller Interferenzkontrast vorgesehen ist.

6. Automatisiertes Zellkultursystem nach Anspruch 5, **dadurch gekennzeichnet, dass** als optische Bilderfassung ein interferometrisches Verfahren vorgesehen ist, bei dem zusätzlich zu einem mikroskopischen Strahlengang ein Referenzstrahl vorgesehen ist, wobei der mikroskopische Strahlengang als Messstrahl mit dem Referenzstrahl überlagerbar ist.

7. Automatisiertes Zellkultursystem nach Anspruch 5 oder 6, **dadurch gekennzeichnet, dass** als optische Bilderfassung eine bildgebende Fluoreszenzoptik vorgesehen ist, mit der eine räumliche Verteilung von Fluoreszenzfarbstoffen in der Zellkultur bestimmbar ist.

8. Automatisiertes Zellkultursystem nach einem der Ansprüche 2 bis 7, **gekennzeichnet durch** eine Einrichtung zur Mittlung der mit der optischen Bilderfassung ermittelten Daten über größere Bereiche der Zellkultur hinweg, insbesondere eine Einrichtung zur Bestimmung der optischen Transmission oder der Fluoreszenz.

9. Automatisiertes Zellkultursystem nach einem der Ansprüche 2 bis 8, **gekennzeichnet durch** einen Zellkulturbehälter, in dem die Zellkultur aufgenommen ist, und eine automatisierte Handhabungseinheit für den Zellkulturbehälter zum Transport der Zellkulturbehälter im automatisierten Zellkultursystem.

10. Automatisiertes Zellkultursystem nach einem der Ansprüche 2 bis 9, **gekennzeichnet durch** elektrische und/ oder elektrochemische Sensoren zur Ermittlung von Prozessbedingungen, insbesondere zur Bestimmung einer lokalen Temperatur, eines pH-Wertes oder einer lonenkonzentration.

11. Automatisiertes Zellkultursystem nach einem der Ansprüche 2 bis 10, **dadurch gekennzeichnet, dass** die Einrichtung zur Regelung von Zellkulturbedingungen dazu vorgesehen ist, den Eingriff direkt im System mit den automatischen aktorischen Elementen zur Handhabung und zum Betrieb der Zellkultur automatisch durchzuführen und/oder eine Meldung nach außen zu geben, insbesondere zusammen mit einem Vorschlag für mögliche Eingriffe an das Bedienpersonal.

12. Automatisiertes Zellkultursystem nach einem der Ansprüche 2 bis 11, **dadurch gekennzeichnet, dass** eine Planung der automatisierten Zellkultivierung bezogen auf einem gewünschten Endzeitpunkt der Produktion erfolgt, wobei ausgehend von dem Zeitpunkt, zu dem die Zelllinien verfügbar sein sollen, eine rückwärtige Planung durchgeführt wird, wann Medienwechsel und Passagen durchgeführt werden sollen.

13. Automatisiertes Zellkultursystem nach einem der Ansprüche 2 bis 12, **dadurch gekennzeichnet, dass** die Systemkomponenten in einer hohen hygienischen Qualität ausgeführt sind, wobei eine Oberflächenrauigkeit kleiner als 20 µm ist, und wobei insbesondere Dichtungselemente aus PTFE oder ähnlichen Materialien ausgebildet sind.

## Claims

1. a) Method of cultivating a cell culture in an automated cell culture system, wherein data concerning the condition of cells are acquired in the cell culture and at least one culturing condition is regulated in accordance with the acquired condition of the cells in the cell culture, and/or at least one operating step determined to be necessary in the cell cultivation process is initiated or carried out, wherein data and/or protocols acquired with the cell culture for the cultivation of cell cultures are stored and can be called in,
b) an apparatus for regulating cell culturing conditions is provided for evaluating a present condition of the cell culture on the basis of the data acquired with the cell culture and stored protocols of earlier cultivations for the cultivation of the present cell cultures, wherein this evaluation is the basis for a determination of a possible intervention in the culturing conditions,
c) at least a part of the cells in the cell culture is analysed with an imaging method, and at least one parameter for the identification of the condition of the cell culture is determined by the evaluation program or a classification of the cells is performed,
d) wherein for the regulation of the culturing conditions, at least one process parameter for the cell cultivation corresponding to the classification performed by the evaluation program is influenced by a regulation program and
the cell culture is transported in a cell culture vessel by an automated handling apparatus in the automated cell culture system,
e) wherein an image analysis program part of the software program for the identification of cells can be trained by the user,
f) wherein in a training phase, some of the images can be marked as examples of structures to be identified, wherein the image analysis program part for these images calculates a set of features and determines those features which characterize the selected examples and determines criteria which discriminate the structures searched for from other structures in the feature space, and wherein, after the termination of the training phase, these calculated parameters are used to automatically identify the desired structures in further images and
g) wherein the user makes, with reference to images, a classification of cell types or of defined conditions of the culture, which can subsequently be identified by the image processing during the automatic cultivation.

2. a) Automated cell culture system with at least one actoric element for handling a cell culture, at least one sensory element for acquiring data concerning the condition of the cells in the cell culture, an apparatus for evaluating the data concerning the condition of the cells, and an apparatus for regulating cell culturing conditions and/or for initiating and/or carrying out further operating steps in the cultivation, for carrying out the method according to claim 1,
b) wherein as sensory elements, an optical imaging microscopic method for the two- and/or three-dimensional representation of the cells is provided,
c) a storage means by which data and/or protocols acquired with the cell culture for the cultivation of cell cultures can be stored and called in,
d) an apparatus for regulating cell culturing conditions is provided for evaluating a present condition of the cell culture on the basis of the data acquired with the cell culture and stored protocols of earlier cultivations for the cultivation of the present cell cultures, wherein this evaluation is the basis for a determination of a possible intervention in the culturing conditions, and
e) the apparatus for regulating cell culturing conditions is provided for carrying out the intervention directly in the system with the automatic actoric elements for the handling and for the operation of the cell culture, and/or for giving a message to the exterior,
f) wherein the apparatuses for evaluating the data concerning the condition of the cells, and an apparatus for regulating cell culturing conditions and/or for initiating and/or carrying out further operating steps is designed as a software program for the evaluation of image data and sensor signals and for the control of the whole process,
g) wherein an image analysis program part of the software program for the identification of cells can be trained by the user,
h) wherein in a training phase, some of the images can be marked as examples of structures to be identified, wherein the image analysis program part for these images calculates a set of features and determines those features which characterize the selected examples and determines criteria which discriminate the structures searched for from other structures in the feature space, and wherein, after the termination of the training phase, these calculated parameters are used to automatically identify the desired structures in further images, and
i) wherein a user makes, with reference to images, a classification of cell types or of defined conditions of the culture, which can subsequently be identified by the image processing during the automatic cultivation.

3. Automated cell culture system according to claim 2, **characterized in that** the actoric element for handling the cell culture vessels, the cell suspensions, the fluorescent dyes and/or the nutrient media is provided.

4. Automated cell culture system according to claim 2 or 3, **characterized by** a conditioned vertical circulation air unit with defined environmental conditions.

5. Automated cell culture system according to one of the claims 2 to 4, **characterized in that** as optical image acquisition, an imaging microscopic method, in particular transmitted light, phase contrast or differential interference contrast, are provided.

6. Automated cell culture system according to claim 5, **characterized in that,** as optical image acquisition, an interferometric method is provided in which, in addition to a microscopic beam path, a reference beam is provided, wherein the microscopic beam path as measurement beam can be superposed by the reference beam.

7. Automated cell culture system according to claim 5 or 6, **characterized in that** as optical image acquisition, imaging fluorescent optics is provided by which a spatial distribution of fluorescent dyes in the cell culture can be determined.

8. Automated cell culture system according to one of the claims 2 to 7, **characterized by** an apparatus for averaging the data acquired with the optical image acquisition over relatively larges areas of the cell culture, in particular an apparatus for the determination of the optical transmission or fluorescence.

9. Automated cell culture system according to one of the claims 2 to 8, **characterized by** a cell culture vessel, carrying the cell culture, and an automated handling apparatus for the cell culture vessel for transporting the cell culture vessel in the automated cell culture system

10. Automated cell culture system according to one of the claims 2 to 9, **characterized by** electrical and/or electrochemical sensors for the determination of process conditions, in particular for the determination of a local temperature, a pH value or an ion concentration.

11. Automated cell culture system according to one of the claims 2 to 10, **characterized in that** the apparatus for the regulation of cell culturing conditions is provided for automatically carrying out the intervention directly in the system with the automatic actoric elements for the handling and for the operation of the cell culture, and/or for giving a message to the exterior to the operators, in particular together with a suggestion for possible interventions.

12. Automated cell culture system according to one of the claims 2 to 11, **characterized in that** a planning of the automated cell cultivation is made with reference to a desired end time of the production, wherein, starting from the point of time at which the cell line are to be available, a backward planning is made stating when a change of the medium and passages are to be performed.

13. Automated cell culture system according to one of the claims 2 to 12, **characterized in that** the system components are designed with a high hygienic quality, wherein a surface roughness is smaller than 20 µm, and wherein in particular sealing elements are made of PTFE or similar materials.

## Revendications

1. a) Procédé de culture d'une culture cellulaire dans un système de culture cellulaire automatisé, dans lequel des données d'état de cellules au sein de la culture cellulaire sont acquises et au moins une condition de culture est régulée en fonction de l'état acquis des cellules au sein de la culture cellulaire et/ou au moins une étape de procédé identifiée comme nécessaire est initialisée ou mise en oeuvre au sein de la culture cellulaire, dans lequel des données acquises au niveau de la culture cellulaire et/ou des protocoles de culture destinés à la culture des cellules sont mémorisé(e)s et peuvent être récupéré(e)s,
b) un dispositif de régulation des conditions de culture cellulaire est prévu pour évaluer un état actuel de la culture cellulaire sur la base des données acquises au niveau de la culture cellulaire et des protocoles enregistrés de cultures précédentes en vue de cultiver les cultures cellulaires actuelles, dans lequel ladite évaluation sert de base pour déterminer une éventuelle intervention dans les conditions de culture,
c) au moins une partie des cellules de la culture cellulaire est examinée à l'aide d'un procédé d'imagerie par microscopie optique, et au moins un paramètre d'identification de l'état de la culture cellulaire est déterminé grâce à un programme d'évaluation ou une classification des cellules est réalisée,
d) dans lequel au moins un paramètre de procédé destiné à la culture de cellules est influencé par un programme de régulation conformément à la classification réalisée par le programme d'évaluation, afin de réguler les conditions de culture, et la culture cellulaire est transportée au sein du système de culture cellulaire automatisé dans un conteneur de culture cellulaire au moyen d'une unité de manipulation automatisée,
e) dans lequel une partie de programme d'analyse d'image d'un programme logiciel peut être formée par l'utilisateur à l'identification des cellules,
f) dans lequel, dans une phase de formation, certaines des images sont marquées comme étant des exemples de structures à identifier, et la partie de programme d'analyse d'image calcule un ensemble d'attributs pour lesdites images et détermine les attributs qui caractérisent les exemples sélectionnés et détermine aussi des critères qui, au sein de l'espace d'attributs, différencient les structures recherchées des autres structures, et dans lequel, à l'issue de la phase de formation, lesdits paramètres calculés sont utilisés pour identifier de manière automatique les structures souhaitées dans d'autres images, et
g) dans lequel l'utilisateur effectue à l'aide d'images une classification de types de cellules ou d'états définis de la culture qui sont ensuite identifiés par un traitement d'image pendant la culture automatisée.

2. a) Système de culture cellulaire automatisé muni d'au moins un élément d'actionnement pour la manipulation d'une culture cellulaire, d'au moins un élément fonctionnant à la manière d'un capteur pour l'acquisition de données d'état des cellules au sein de la culture cellulaire, d'un dispositif d'évaluation des données d'état des cellules et d'un dispositif de régulation des conditions de culture cellulaire et/ou d'initialisation et/ou de mise en oeuvre d'autres étapes de procédé lors de la culture, afin de mettre en oeuvre le procédé selon la revendication 1,
b) dans lequel une capture d'image par microscopie optique est prévue, en tant qu'élément fonctionnant à la manière d'un capteur, pour la représentation bidimensionnelle ou tridimensionnelle des cellules,
c) un dispositif formant mémoire avec lequel des données acquises au niveau de la culture cellulaire et/ou des protocoles destinés la culture des cultures cellulaires peuvent être mémorisé(e)s et récupéré(e)s,
d) un dispositif de régulation des conditions de culture cellulaire est prévu pour évaluer un état actuel de la culture cellulaire sur la base des données acquises au niveau de la culture cellulaire et des protocoles mémorisés de cultures précédentes pour la culture des cultures cellulaires actuelles, dans lequel ladite évaluation sert de base pour déterminer une éventuelle intervention dans les conditions de culture, et
e) dans lequel le dispositif de régulation des conditions de culture cellulaire est prévu pour intervenir de manière automatique directement au sein du système avec les éléments d'actionnement automatiques afin de manipuler et exploiter la culture cellulaire, et/ou pour transmettre un message vers l'extérieur,
f) dans lequel les dispositifs d'évaluation des données d'état des cellules et de régulation des conditions de culture cellulaire et/ou d'initialisation et/ou d'exécution d'autres étapes de procédé sont réalisés sous forme de programme logiciel destiné à évaluer des données d'image et à commander l'ensemble du déroulement,
g) dans lequel une partie de programme d'analyse d'image du programme logiciel peut être formée par l'utilisateur à l'identification des cellules,
h) dans lequel, dans la phase de formation, certaines des images peuvent être marquées comme étant des exemples de structures à identifier, dans lequel la partie de programme d'analyse d'image calcule un ensemble d'attributs pour lesdites images et détermine les attributs qui caractérisent les exemples sélectionnés et détermine aussi des critères qui différencient les structures recherchées des autres structures, et dans lequel, à l'issue de la phase de formation, lesdits paramètres calculés sont utilisés pour identifier de manière automatique les structures souhaitées dans d'autres images, et
i) dans lequel un utilisateur effectue à l'aide d'images une classification de types de cellules ou d'états définis de la culture qui sont ensuite identifiés par un traitement d'image pendant la culture automatisée.

3. Système de culture cellulaire automatisé selon la revendication 2, **caractérisé en ce que** l'élément actionneur est prévu pour manipuler les récipients de culture cellulaire, les suspensions cellulaires, les colorants fluorescents et/ou les milieux nutritifs.

4. Système de culture cellulaire automatisé selon la revendication 2 ou 3, **caractérisé par** un banc à circulation verticale climatisé présentant des conditions environnementales définies.

5. Système de culture cellulaire automatisé selon l'une quelconque des revendications 2 à 4, **caractérisé en ce qu'**une acquisition optique d'image, en particulier sous lumière transmise, à contraste de phase ou à contraste interférentiel différentiel est prévue en tant que procédé microscopique.

6. Système de culture cellulaire automatisé selon la revendication 5, **caractérisé en ce qu'**un procédé interférométrique est prévu en tant qu'acquisition optique d'image, dans lequel un faisceau de référence est prévu en plus d'un trajet de faisceau microscopique, dans lequel le trajet de faisceau microscopique peut être superposé au faisceau de référence en tant que faisceau de mesure.

7. Système de culture cellulaire automatisé selon la revendication 5 ou 6, **caractérisé en ce qu'**une optique de fluorescence d'imagerie avec laquelle une répartition spatiale des colorants fluorescents au sein de la culture cellulaire peut être déterminée est prévue en tant qu'acquisition optique d'image.

8. Système de culture cellulaire automatisé selon l'une quelconque des revendications 2 à 7, **caractérisé par** un dispositif de moyennage des données déterminées lors de l'acquisition optique d'image sur de plus grandes régions de la culture cellulaire, en particulier un dispositif de détermination de la transmission optique ou de la fluorescence.

9. Système de culture cellulaire automatisé selon l'une quelconque des revendications 2 à 8, **caractérisé par** un conteneur de culture cellulaire au sein duquel est accueillie la culture cellulaire, et une unité de manipulation automatisée destinée au conteneur de culture cellulaire en vue du transport du conteneur de culture cellulaire au sein du système de culture cellulaire automatisé.

10. Système de culture cellulaire automatisé selon l'une quelconque des revendications 2 à 9, **caractérisé par** des capteurs électriques et/ou électrochimiques pour la détermination des conditions de procédé, en particulier pour la détermination d'une température locale, d'une valeur de pH ou d'une concentration en ions.

11. Système de culture cellulaire automatisé selon l'une quelconque des revendications 2 à 10, **caractérisé en ce que** le dispositif de régulation des conditions de culture cellulaire est prévu pour intervenir de manière automatique directement au sein du système avec les éléments d'actionnement automatiques afin de manipuler et exploiter la culture cellulaire, et/ou pour transmettre un message vers l'extérieur, en particulier en commun avec une suggestion d'intervention éventuelle présentée au personnel d'exploitation.

12. Système de culture cellulaire automatisé selon l'une quelconque des revendications 2 à 11, **caractérisé en ce qu'**une planification de la culture cellulaire automatisée est effectuée par rapport à une heure de fin souhaitée de la production, dans lequel une rétroplanification des moments où des remplacements de milieu et des passages doivent être effectués est réalisée en partant du moment où les lignées cellulaires doivent être disponibles,

13. Système de culture cellulaire automatisé selon l'une quelconque des revendications 2 à 12, **caractérisé en ce que** les composants du système sont réalisés avec une qualité d'hygiène élevée, dans lequel une rugosité de surface est inférieure à 20 µm, et dans lequel en particulier des éléments d'étanchéité sont réalisés à partir de PTFE ou de matériaux similaires.
